# EUROPEAN PATENT SPECIFICATION

(11) **EP 0 758 219 B1**
(45) Date of publication and mention of the grant of the patent: **26.08.1998**
(21) Application number: 95918801.2
(22) Date of filing: 07.04.1995
(51) Int. Cl.: A61F 13/00, A61F 13/48

(54) **A METHOD FOR PRODUCING AN ABSORBENT STRUCTURE AND AN ABSORBENT WOUND DRESSING WHICH INCLUDES AN ABSORBENT STRUCTURE PRODUCED IN ACCORDANCE WITH THE METHOD**
VERFAHREN ZUR HERSTELLUNG EINER ABSORBIERENDEN STRUKTUR UND EIN ABSORBIERENDER WUNDVERBAND MIT EINER ABSORBIERENDEN STRUKTUR, HERGESTELLT DURCH DIESES VERFAHREN
PROCEDE DE PRODUCTION D'UNE STRUCTURE ABSORBANTE ET PANSEMENT ABSORBANT COMPORTANT UNE STRUCTURE ABSORBANTE PRODUITE SELON LEDIT PROCEDE

(30) Priority: 04.05.1994 SE 9401543
(43) Date of publication of application: 19.02.1997
(73) Proprietor: SCA Mölnlycke AB, 405 03 Göteborg (SE)
(72) Inventor: FABO, Thomas, S-435 41 Mölnlycke (SE); KUUSE, Staffan, S-430 63 Hindas (SE)
(74) Representative: Larsson, Karin
(86) International application number: SE9500379
(87) International publication number: WO9530394

(56) References cited:
- GB-A- 2 055 297
- GB-A- 2 115 702
- SE-T- 444 073

## Description

### Background

The present invention relates to a method for producing an absorbent structure in an absorbent article, such as a wound dressing and like articles.

Many different embodiments of absorbent articles of this kind are known to the art. Conventionally, the absorbent bodies used in these products are produced by dry-defibering cellulose pulp contained, for instance in reels, bales or in sheet form, and converting the pulp to a pulp mat in a fluffed state, sometimes while admixing so-called superabsorbents, which are polymers capable of absorbing several times their own weight in water or body fluid. The pulp body is often compressed, partly to reduce the bulk of the body and partly to increase its liquid wicking capacity.

The absorbent body may also include other constituents, for instance constituents which will improve its liquid acquisation properties or its liquid wicking properties, or to increase its coherency and its ability to resist deformation in use.

The rear side of an absorbent article such as a wound dressing is not normally impervious to liquid, but is slightly liquid-permeable so that the wound can breathe. The bacterial growth rate in a product which includes an impervious rear side is normally much higher than in a product where the rear side is manufactured from a material which has some permeability. Products whose rear sides are made, for instance, from a nonwoven material that has been made hydrophobic are therefore more suitable as wound dressings, since there is less risk of the wound being infected. One serious problem with wound dressings of this kind, however, is that the products often leak long before their total absorption capacity has been utilized to the full. Among other things, this is because the body fluid discharged is unable to penetrate into the absorbent material and spread to hitherto unused parts of the product, but instead leaks from the sides of the dressing or through its rear side. The ability of the materials used to disperse the absorbed liquid or fluid throughout the whole of the absorbent body is thus of great importance.

So-called rewetting is another problem, by which is meant that body fluid that has already been observed is pressed back against the wearer's skin when the absorbent body is subjected to an external force, or by gravity. It is generally desired that the surface of the article which lies proximal to the user's skin shall be relatively dry. A thin wound dressing is desired in many instances, so that the dressing can be worn as comfortably as possible.

A very large part of the production plant used by manufacturers in the production of the aforesaid sanitary articles is comprised of defibration equipment, pneumatic conveyor systems and mat-forming equipment. This equipment is also a serious source of production plant malfunctioning and manufacturing errors. This equipment is often followed by equipment for compressing the finished pulp mat or the finished sanitary product.

It is known from European Patent EP 0,444,073 that a pulp web intended for later defibration, so-called dry-formed reel pulp, can be produced with the aid of dry-forming processes. Flash-dried paper pulp fibres, which may be comprised of thermomechanical pulp, chemi-thermomechanical pulp CTMP, or chemical paper pulp, sulphite pulp or sulphate pulp having a dryness of about 80%, are delivered by means of an air stream in a controlled flow to a forming head mounted above a wire and thereby formed into a web having a surface weight of 300-1,500 g/m² and a density of 550-1,000 kg/m³. The vehicle air is removed by suction through a suction box mounted beneath the wire. The web shall have a moisture content of 50-30%.

The web is pre-pressed with the intention of reducing the bulk of the web slightly, prior to finally compressing the web to a density of 550-1,000 kg/m³. The pressed web is sufficiently strong to enable it to be rolled-up or handled in sheet form for storage and transportation purposes. The pulp can be readily defibered and is intended to be transferred in the form of fluff for use in the manufacture of absorbent bodies in diapers, sanitary napkins and like products. A similar method is known from GB-A-2 115 702.

Another method of manufacturing an absorbent structure is described in EP 0,122,042, wherein a mixture of hydrophilic fibres and water-insoluble hydrogel particles are air-laid to form a web and compressed to a density of about 0.15-1.0 g/cm³. This method, however, includes a number of production stages in which cellulose pulp, normally contained in the form of bales or sheet reels, is first defibered in a hammer mill to obtain cellulose fibres in the form of fluff pulp, whereafter the fibres are laid on a forming belt and formed into the absorbent structure, which is then compressed. These production stages make this process relatively complicated and expensive.

In the case of certain sanitary article production applications, it is suitable to soften dry-formed reel pulp prior to its use as absorbent material. This softening process does not effect the earlier mentioned good absorbent properties, wicking properties and swelling properties of the pulp to any great extent. One method of softening an absorbent sheet is described in EP 0,360,472, according to which the compressed absorbent material is worked between partially cutting rolls and therewith softened. However, this method results in a reduction in the strength of the softened material, among other things.

### Summary of the Invention

The object of the present invention is to provide an absorbent structure in an absorbent article of the kind defined in the introduction which will exhibit good absorption properties, primarily with regard to its ability to disperse liquid in the material. The material will preferably also have low rewetting tendencies and shall be capable of being made very thin. Another object is to provide a simplified method of producing absorbent articles of the kind defined in the introduction. This has been realized in accordance with the invention with a method of manufacture in which material in particle form and including 80-100 percent, preferably at least 85 percent and more preferably at least 90 percent flash-dried cellulose fibres is dry-formed to a web that has a surface weight of 100-1,000 g/m² and compressed to a density of 0.1-0.5 g/cm³, and in which the web is incorporated as an absorbent structure in an absorbent article without subsequent defibration and fluff-forming, wherein the absorbent article has a rear side which is made of a liquid-repelling but vapour-permeable material.

### Brief Description of the Drawings

The invention will now be described in more detail with reference to a number of exemplifying embodiments thereof and also with reference to the accompanying drawings, in which
Figure 1 illustrates the absorption properties of a dry-formed CTMP-material after being worked between rolls which are spaced at different distances apart. Conventionally formed and compressed pulp mats of CTMP and chemical pulp respectively are used as references.
Figure 2 illustrates the absorption properties of a softened dry-formed CTMP-material. Conventionally formed and compressed pulp mats of CTMP and chemical pulp respectively are used as references.
Figure 3 illustrates the rewetting tendency of non-softened and softened absorbent structures having a core which was manufactured from dry-formed CTMP, with and without a superabsorbent admixture, the liquid absorbed being blood.
Figure 4 illustrates the rewetting tendency of a complete absorbent article having a core manufactured from dry-formed CTMP, the liquid absorbed being blood. Conventionally manufactured products of corresponding composition are used as a reference.
Figure 5 illustrates the growth rate of different microorganisms in test products whose rear sides were made of nonwoven material that had been made hydrophobic and an impervious polyethylene film, and whose rear sides only were made of nonwoven material that had been made hydrophobic, respectively.
Figure 6 illustrates schematically the construction of an inventive absorbent article.
Figure 7 is a cross-sectional, schematic view of the material in an unsoftened state.
Figure 8 is a cross-sectional, schematic view of the material in a softened state.

### Description of the Invention

As before mentioned, important properties of the material used in the manufacture of a sanitary product, such as an absorbent wound dressing or the like, are its absorption capacity, absorption rate, wicking ability, fluid drainage ability, coherency, rewetting tendencies, softness and smoothness. The liquids in question are blood and wound fluids.

The object of the present invention is to provide in an absorbent article, such as a wound dressing and like articles, an absorbent structure which has extremely good absorption properties, primarily with regard to its ability to spread liquid throughout the material. The material will also preferably have low rewetting tendencies and shall be capable of being made very thin and pliant. It is also an object to simplify the manufacturing process. A finished absorbent material in reel form that can be used without defibering the material would reduce partially the need of the earlier mentioned defibration equipment, pneumatic conveyor system and mat-forming equipment, and consequently there is a desire for absorbent material ot this nature.

The aforesaid objects of the invention have been achieved with a method of manufacture in which material in particle form and comprised of 80-100 percent by weight, preferably at least 85 percent by weight and more preferably at least 90 percent by weight flash-dried cellulose fibres is dry-formed to a web having a surface weight of 100-1,000 g/m² and compressed to a density of 0.2-0.5 g/cm³, and in which the web is incorporated as an absorbent structure in an absorbent article without subsequent defibration and fluffing, and in which the rear side of the absorbent article is comprised of a liquid-repelling but vapour-permeable material.

When practicing the present invention, there is used a dry-formed product manufactured from mechanical pulp or chemi-thermomechanical pulp (CTMP), or a corresponding product manufactured from sulphite pulp or sulphate pulp, so-called chemical cellulose pulp. Cellulose fibres that have been stiffened chemically may also be used. The dry-formed product may also incorporate other particulate constituents, for instance superabsorbents, thermoplastic binding fibres and other types of fibre.

Dry-formed reel pulp has very good absorption, wicking and swelling properties when in an unworked state, and it has been found possible to use the material as absorption material in sanitary articles without directly defibering the material. It has also been found that because of the good wicking properties of dry-formed reel pulp, it is possible to use on the rear side of the article a material which is more permeable than the absorption materials used conventionally in the absorbent structure. This is beneficial to the treatment and healing of wounds. To enable the material to be used in absorbent wound dressings, it is suitable to soften the material in some way or another. One method of softening the material is described below.

Dry-formed reel pulp has good coherency, which means that when superabsorbent materials are used they are able to bind effectively to the absorbent structure and will not therefore be spread in the surroundings of the plant machinery during the continued manufacture of the absorbent sanitary articles.

Dry-formed cellulose pulp can be produced, for instance, by forming a web of flash-dried paper pulp fibres in accordance with the method described in EP 0,444,073.

Cellulose pulp fibres have a so-called curl value which defines the curl of the fibre. Curl value can be measured by the method described by B.D. Jordan, N.G. Nguyen in Papper och Trä 4/1986, page 313.

### Softening of the Material

The material can be softened to an extent which renders it extremely suitable for use as absorption material in absorbent wound dressings, by working dry-formed reel pulp between wave-shaped rolls for instance. The material can be softened to mutually different extents, by working the material between different types of rolls and at different roll spacings or nip sizes. The aforesaid good absorption properties are not influenced to any great extent by the softening process.

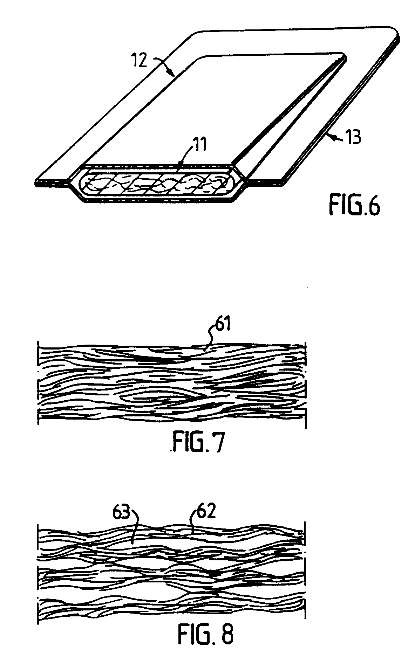

In the softening process, the material is delaminated so as to form a plurality of partially separated thin fibre layers. This is illustrated schematically in Figures 7 and 8. The material 61 that has not been softened will normally have a uniform high density throughout its entire thickness. The softening process delaminates the material and forms a plurality of partially separated 63 fibre layers 62. This softening and delaminating process reduces the total density of the material to some extent, while essentially retaining the original density in each individual layer. As a result of retaining a very high density in the individual layers, the good wicking properties of the material are retained despite the increase in bulk that occurs in conjunction with softening the material. The total bulk is increased by up to 300%, normally 1-100% by the softening process, depending on the method applied and the extent to which the material is softened.

The aforedescribed method of softening the material shall be seen merely as an example, and other methods may also be applied to achieve corresponding results. Examples of other methods which may be used in this regard are softening with the aid of ultrasonic processes, microwaves, moisturizing, or with the aid of chemical additives.

### Investigating the Properties of the Material

Different test equipment were used to evaluate the absorption properties of the material, as described below.

### Method 1 Absorption Properties Up a Sloping Surface

A rectangular sample body was punched from the material and a line was drawn across the body at a point 11 cm from one short end thereof. A liquid-containing vessel was placed adjacent laboratory scales and both the scales and the liquid-containing vessel were adjusted to a horizontal position. A plexiglass plate was placed on the scales at an angle of 30°, with one free edge of the plate extending down into the vessel. A line had been drawn across the plate at a point 11 cm from the lower edge of said plate. Sample liquid (0.9% NaCl-solution) was poured into the vessel until 20 mm of the plexiglass plate was located beneath the surface of the liquid. The sample body was affixed to the plate, so that the line on the sample body coincided with the line on the plate while folding away the lower part of the sample body out of contact with the sample liquid. A clock was started at the same time as the sample body was placed on the plexiglass plate. The sample body was placed on the plate so as to extend into the sample solution to the same depth as the plexiglass plate. The increase in weight of the sample body was recorded with time.

### Method 2 Determining Blood Absorption and Rewetting

A sample body measuring 65 x 200 mm was punched from the material. 5 ml of sample liquid (0.9% NaCl-solution) were applied to the wetting point of the sample body. Dispersion of the liquid was measured after about 30 minutes. A further 5 ml of sample liquid (0.9% NaCl-solution) were added and dispersion of the liquid was measured after about a further 30 minutes. After this last addition, eight filter papers were placed over the wetting point and a load of 4.875 kg was applied for a period of 15 seconds. The filter papers were weighed before and after applying the load and the rewetting was recorded.

### Method 3 Determining Bacterial Growth

Two types of test product were produced. The test products comprised conventionally an absorbent body of defibered cellulose pulp measuring 10 x 10 cm and had a surface weight of 300 g/m² and a density of 0.15 g/m³, and was enclosed between a liquid-permeable top sheet made of soft nonwoven material, 20 g/m², and a backing sheet. The nonwoven material and the backing sheet had parts which extended beyond the absorbent body and were joined together at these parts. Sample B had a material at the rear side that had been made hydrophobic, and sample A had an impervious polyethylene film between the nonwoven material that had been made hydrophobic and the absorbent body. Bacteria suspensions containing **Staphylococcus aureus, Pseudomonas aeruginosa,** and **Clostridium sporogenes** respectively and a yeast suspension containing **Candida albicans** was prepared, and from each suspension, 10 ml dosages containing about 1,000 CFU per dosage of the bacteria suspension of **Staphylococcus aureus,** and **Clostridium sporogenes**, respectively, and about 500 CFU per dosage of the bacterial suspension of **Pseudomonas aeruginosa**, and about 50 CFU per dosage of the yeast suspension of **Candida albicans** were taken. Dosages of 10 ml of each of the suspensions was added to each test product of the types A and B respectively, whereafter these products were incubated at 30°C for 40 hours. The number of bacteria in the samples was then measured.
- Sample A: Test product having a rear side made of nonwoven material that has been made hydrophobic and impervious polyethylene film.
- Sample B: Test product having a rear side made of nonwoven material that has been made hydrophobic.

### Test Results

### Softening

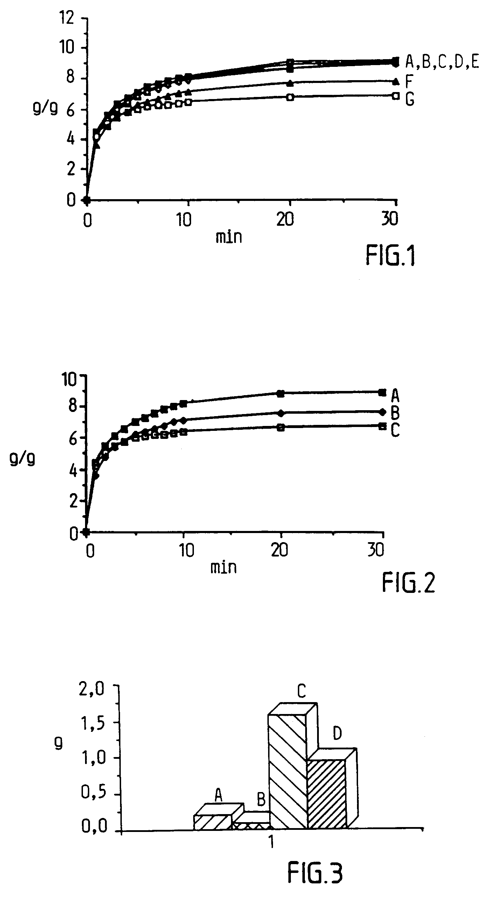

A material was tested under different softening conditions, with the intention of establishing how the material was affected by being softened between softening rollers at different roll spacings. When softening the material, a suitable roll spacing is 0.7-2.4 mm in the case of a dry-formed CTMP-material having a surface weight of 900 g/m² and a density of 0.63 g/cm³ for instance, and the material is not affected to any appreciable extent by different roll spacings within this range. Figure 1 illustrates the absorption properties at different roll spacings. The results are measured in accordance with Method 1.
- A: Inventive material, roll spacing 1.7 mm.
- B: Inventive material, roll spacing 2.0 mm.
- C: Inventive material, roll spacing 2.4 mm.
- D: Inventive material, roll spacing 2.0 mm, softened twice.
- E: Inventive material, roll spacing 2.0 mm, softened four times.
- F: CTMP-pulp, density 0.125 g/cm³.
- G: Chemical sulphate pulp, density 0.125 g/cm³.

### Absorption Properties of Absorbent Structures

Figure 2 illustrates the absorption properties of a CTMP-material according to the invention having a surface weight of 900 g/m² and a density of 0.63 g/cm³ compared with corresponding pulp cores of conventionally defibered and mat-formed CTMP and corresponding chemical pulp. The absorption capacity in the absence of superabsorbent material is about 9 g liquid per g of absorbent material. The results are measured according to Method 1.
- A: Inventive material.
- B: CTMP-pulp, density 0.125 g/cm³.
- C: Chemical sulphate pulp, density 0.125 g/cm³.

### Determining Rewetting, Specific for Blood Absorption

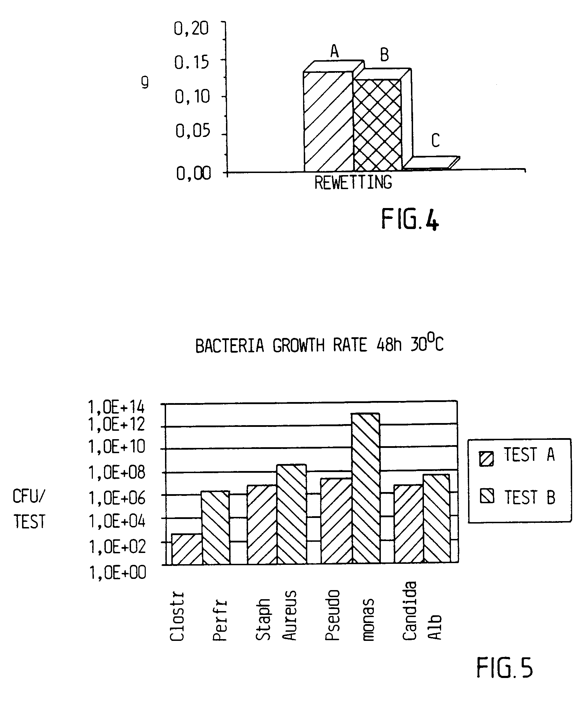

With regard to blood absorption, products comprised of softened CTMP-material according to the invention were found to have better rewetting values than products that had not been softened. The results also show that blood absorption products which contain no superabsorbent material exhibit lower rewetting values than material that contain superabsorbent material. Material which contains no superabsorption material also disperses blood much better. The results are shown in Figure 3 and Figure 4. The reference products comprised two different products that occur frequently on the market. The results are measured according to Method 2. In order to achieve this effect, it is necessary for at least one layer of the pulp mat to be free from superabsorbent material, although this does not exclude the presence of such material in other parts of the absorbent article.

### Figure 3

- A: Inventive material, 350 g/m².
- B: Inventive material, 350 g/m², softened.
- C: Inventive material, 350 g/m² + 5% superabsorbent.
- D: Inventive material, 350 g/m² + 5% superabsorbent, softened.

### Figure 4

- A: Reference product 1.
- B: Reference product 2.
- C: Product containing inventive material.

### Bacteria Growth

The growth of bacteria in the samples comprising an impervious rear side is much greater than in the samples whose rear sides comprised hydrophobic nonwoven material, for all tested microorganisms. The results are shown in Figure 5. The results have been measured according to Method 3. The results show that products whose rear sides are comprised solely of hydrophobic nonwoven material are more suited for use as wound dressings, since they present less risk of wound infection.
- Sample A: Test product having a rear side made of nonwoven material that has been made hydrophobic and impervious polyethylene film.
- Sample B: Test product having a rear side made of nonwoven material that has been made hydrophobic.

### Network Strength

The mat strength of dry-formed reel pulp is normally sufficient for those product applications intended here. Should the network strength prove insufficient in certain product applications, the network strength can be increased by reinforcing the structure in an appropriate manner, by adding reinforcement fibres, binding fibres or binding agent to the cellulose fibre mixture. The network strength may also be improved by inserting into the absorbent structure a reinforcement layer of plastic, nonwoven, net or threads for instance, or by fastening a reinforcing sheet or a surface sheet to one or both sides of the material.

### Density and Surface Weight

The softened pulp mat is still very thin, and consequently it is unnecessary in many cases to further compress the mat prior to its use in an absorbent article. A suitable density is 0.10-0.50 g/cm³, preferably 0.20-0.45 g/cm³ and more preferably 0.30-0.40 g/cm³. A suitable surface weight is 100-1,000 g/m², preferably 150-800 g/m² and more preferably 200-700 g/m². A Mitutoyo thickness meter was used to measure the thickness of the material when calculating the density.

### Description of an Exemplifying Embodiment

Figure 6 illustrates an exemplifying embodiment of an inventive wound dressing. The wound dressing comprises conventionally an absorbent body 11 which is enclosed between a liquid-permeable top sheet 12, suitably comprised of soft nonwoven material, a perforated plastic film or the like, and which lies proximal to the wearer in use, and a liquid-repelling bottom or backing sheet 13. The sheets 12 and 13 have parts which extend beyond the absorbent body 11 and are joined together at these parts. A thin liquid-permeable layer or sheet (not shown), for instance of nonwoven material, may be placed between the top sheet 12 and the absorption body 11. The bottom sheet 13 is comprised suitably of a suitable liquid-repelling material, for instance a nonwoven material that has been made hydrophobic. It will be understood that the top and the bottom sheets may be made of other known materials, within the scope of the invention.

The illustrated absorbent body 11 includes only a single layer. This layer may be comprised of dry-formed material according to the invention and may have a relatively open fibre structure of relatively low density, and may contain 0-10% superabsorbent material. A suitable density range in the case of the absorbent body 11 is 0.20-0.50 g/cm³, while a suitable surface weight is 200-700 g/m². When the absorbent body 11 is comprised of a CTMP-material or some other material that has a yellowish or brownish colour, a covering layer of white chemical pulp may optionally be applied to the absorbent body.

It will be understood that the invention is not limited to the illustrated exemplifying embodiments thereof and that other embodiments can be applied within the scope of the following Claims.

## Claims

1. A method for producing an absorbent structure (11) in an absorbent article such as a wound dressing and like article, wherein the absorbent article has a rear side (13) which is comprised of liquid-repelling but vapour permeable material, **characterized** by dry-forming material in particle form which comprises flash-dried cellulose fibres into a web with a surface weight of 80-1,000 g/m², compressing the web to a density of 0.10-0.50 g/cm³, and incorporating the web as an absorbent structure (11) in an absorbent article without subsequent defibration and fluffing of the web.

2. A method for producing an absorbent structure (11) according to Claim 1, **characterized** by compressing the web to a density of 0.20-0.45 g/cm³, preferably 0.30-0.40 g/cm³.

3. A method for producing an absorbent structure (11) in accordance with any one of the preceding Claims, **characterized** by softening the web mechanically and therewith delaminating said web and forming a plurality of partially separated thin fibre layers prior to incorporating the web in an absorbent article.

4. An absorbent structure (11) consisting of a web of dry-formed material in particle form comprising flash-dried and compressed cellulose fibres and having a surface weight of 80-1.000 g/cm² and a density of 0.10-0.50 g/cm³, where said absorbent structure has not been defibrated or fluffed.

5. An absorbent structure (11) according to Claim 4, **characterized** in that the structure has a surface weight of 150-800 g/m², preferably 200-700 g/m².

6. An absorbent structure (11) according to Claim 4 or 5, **characterized** in that the cellulose fibres are essentially comprised of fibres of chemithermomechanically produced pulp.

7. An absorbent structure (11) according to Claim 6, **characterized** in that the chemithermomechanical pulp fibres have a curl value of 0.20-0.40.

8. An absorbent structure (11) according to any one of Claims 4-7, **characterized** in that at least a part of the fibres are chemically stiffened cellulose fibres.

9. An absorbent structure (11) according to any one of the preceding Claims, **characterized** in that the structure includes at most 20 percent by weight, preferably at most 10 percent by weight and more preferably at most 5 percent by weight superabsorbent material, calculated on the total weight of the structure in a dry state.

10. An absorbent structure (11) according to any one of the preceding Claims, **characterized** in that the structure includes reinforcement agent, for instance binder, reinforcing fibres or thermoplastic binding fibres.

11. An absorbent structure (11) according to any one of the preceding Claims, **characterized** in that the structure includes a reinforcing layer of nonwoven, tissue, plastic or net, for instance.

12. An absorbent article such as a wound dressing and like article comprising a liquid-permeable top sheet (12), a liquid-repelling but vapour-permeable bottom or backing sheet (13) and an absorbent body (11) enclosed between said sheets, **characterized** in that the absorbent body (11) includes an absorbent structure (11) according to any one of Claims 4-11.

13. An absorbent article according to Claim 12, **characterized** in that the absorbent structure (11) includes essentially fibres of chemithermomechanically produced pulp; and in that the structure includes between 0-10 percent by weight superabsorbent material, calculated on the total weight of the structure in a dry state.

14. An absorbent article according to any one of Claims 12 and 13, **characterized** in that the absorbent structure (11) is provided with a fully covering layer of chemical pulp on one side thereof.

## Patentansprüche

1. Verfahren zum Herstellen einer absorbierenden Struktur (11) in einem absorbierendem Artikel, wie zum Beispiel ein Wundverband und dergleichen mehr, bei dem der absorbierende Artikel eine Rückseite (13) besitzt, die aus flüssigkeitsabstoßendem, aber dampfdurchlässigem Material besteht, **gekennzeichnet durch** Dryforming von in Partikelform vorliegendem Material, das schnellgetrockenete Zellulosefasern umfaßt, in eine Bahn mit einem Flächengewicht von 80-1000 g/m², Zusammenpressen der Bahn auf eine Dichte von 0,10-0,50 g/m³ und Einfügen der Bahn als absorbierende Struktur (11) in einen absorbierenden Artikel ohne nachfolgende Zerfaserung und Fluff-Bildung.

2. Verfahren zum Herstellen einer absorbierenden Struktur (11) nach Anspruch 1, **gekennzeichnet durch** Zusammenpressen der Bahn auf eine Dichte von 0,20-0,45 g/m³, vorzugsweise 0,30-0,40 g/m³.

3. Verfahren zum Herstellen einer absorbierenden Struktur (11) nach einem der vorhergehenden Ansprüche, **gekennzeichnet durch** mechanisches Weichmachen der Bahn und hiermit Delaminieren der Bahn und Formen einer Vielzahl teilweise separierter dünner Faserschichten vor dem Einfügen der Bahn in, einen absorbierenden Artikel.

4. Absorbierende Struktur (11), bestehend aus einer Bahn aus trockengeformtem Material in Partikelform, umfassend schnellgetrockenete und zusammengepresste Zellulosefasern, und ein Flächengewicht von 80-1000 g/m³ und eine Dichte von 0,10-0,50 g/m³ aufweisend, wobei die absorbierende Struktur nicht zerfasert oder geflufft wurde.

5. Absorbierende Struktur (11) nach Anspruch 4, **dadurch gekennzeichnet, daß** die Struktur ein Flächengewicht von 150-800 g/m², vorzugsweise 200-700 g/m², besitzt.

6. Absorbierende Struktur (11) nach Anspruch 4 oder 5, **dadurch gekennzeichnet, daß** die Zellulosefasern im wesentlichen aus Fasern aus chemithermomechanisch hergestelltem Zellstoff bestehen.

7. Absorbierende Struktur (11) nach Anspruch 6, **dadurch gekennzeichnet, daß** die chemithermomechanisch hergestellten Zellstoffasern einen Curl-Wert von 0,20-0,40 haben.

8. Absorbierende Struktur (11) nach einem der Ansprüche 4-7, **dadurch gekennzeichnet, daß** zumindest ein Teil der Fasern chemisch versteifte Zellulossefasern sind.

9. Absorbierende Struktur (11) nach einem der Ansprüche 4-7, **dadurch gekennzeichnet, daß** die Struktur höchstens 20 Gewichtsprozent, vorzugsweise höchstens 10 Gewichtsprozent und bevorzugtermaßen 5 Gewichtsprozent hochsaugfähiges Material beinhaltet, berechnet vom Strukturgesamtgewicht in trockenem Zustand.

10. Absorbierende Struktur (11) nach einem der vorhergehenden Ansprüche, **dadurch gekennzeichnet, daß** die Struktur ein Verstärkungsmittel, beispielsweise ein Bindemittel, Verstärkungsfasern oder thermoplastische Bindefasern, beinhaltet.

11. Absorbierende Struktur (11) nach einem der vorhergehenden Ansprüche, **dadurch gekennzeichnet, daß** die Struktur eine Verstärkungsschicht aus beispielsweise Vlies, Gewebe, Kunststoff oder Netz beinhaltet.

12. Absorbierender Artikel, wie beispielsweise ein Wundverband und dergleichen mehr, umfassend eine flüssigkeitsdurchlässige Oberlage (12), eine flüssigkeitsabstoßende, aber dampfdurchlässige Grund- oder Stützlage (13) und einen zwischen den Lagen eingeschlossenen absorbierenden Körper (11), **dadurch gekennzeichnet, daß** der absorbierende Körper (11) eine absorbierende Struktur nach einem der Ansprüche 4-11 beinhaltet.

13. Absorbierender Artikel nach Anspruch 12, **dadurch gekennzeichnet, daß** die absorbierende Struktur (11) im wesentlichen Fasern aus chemithermomechanisch hergestelltem Zellstoff beinhaltet, und daß die Struktur zwischen 0-10 Gewichtsprozent hochsaugfähigem Material beinhaltet, berechnet vom Gesamtgewicht der Struktur in trockenem Zustand.

14. Absorbierender Artikel nach einem der Ansprüche 12 und 13, **dadurch gekennzeichnet, daß** die absorbierende Struktur (11) auf einer Seite mit einer vollständig bedeckenden Schicht aus chemischem Zellstoff versehen ist.

## Revendications

1. Procédé de production d'une structure absorbante (11) dans un article absorbant comme un pansement ou analogue, dans lequel l'article absorbant a une face arrière (13) qui est composée d'un matériau repoussant les liquides mais perméable à la vapeur, caractérisé par la formation à sec d'un matériau sous forme particulaire qui comprend des fibres de cellulose séchées rapidement pour donner une nappe ayant une masse surfacique de 80 à 1000 g/m², la compression de la nappe jusqu'à une densité de 0,10 à 0,50 g/m³, et l'incorporation de la nappe en tant que structure absorbante (11) dans un article absorbant sans avoir ensuite de défibrage ni de mise sous forme de fluff de la nappe.

2. Procédé de production d'une structure absorbante (11) selon la revendication 1, caractérisé par une compression de la nappe jusqu'à une densité de 0,20 à 0,45 g/cm³ et de préférence de 0,30 à 0,40 g/cm³.

3. Procédé de production d'une structure absorbante (11) selon l'une quelconque des précédentes revendications, caractérisé par un assouplissement mécanique de la nappe et, en même temps, une délamination de ladite nappe et la formation d'une pluralité de minces couches de fibres partiellement séparées avant l'incorporation de la nappe dans un article absorbant.

4. Structure absorbante (11) faite d'une nappe de matériau formé à sec sous forme particulaire, comprenant des fibres de cellulose séchées rapidement et comprimées, ayant une masse surfacique de 80 à 1000 g/m² et une densité de 0,10 à 0,50 g/m³, ladite structure absorbante n'ayant été ni défibrée ni mise sous forme de fluff.

5. Structure absorbante (11) selon la revendication 4, caractérisée en ce que la structure a une masse surfacique de 150 à 800 g/m² et de préférence de 200 à 700 g/m².

6. Structure absorbante (11) selon la revendication 4 ou 5, caractérisée en ce que les fibres de cellulose sont essentiellement faites de fibres de pulpe produite par voie chimio-thermo-mécanique.

7. Structure absorbante (11) selon la revendication 6, caractérisée en ce que les fibres de pulpe chimio-thermo-mécanique ont un degré de frisure compris entre 0,20 et 0,40.

8. Structure absorbante (11) selon l'une quelconque des revendications 4 à 7, caractérisée en ce que une partie au moins des fibres sont des fibres de cellulose rigidifiées chimiquement.

9. Structure absorbante (11) selon l'une quelconque des précédentes revendications, caractérisée en ce que la structure contient au plus 20% en poids, de préférence au plus 10% en poids et mieux encore au plus 5% en poids, d'un matériau superabsorbant, cette valeur étant rapportée au poids total de la structure à l'état sec.

10. Structure absorbante (11) selon l'une quelconque des précédentes revendications, caractérisée en ce que la structure contient un agent de renfort, par exemple un liant, des fibres de renfort ou des fibres de liaison thermoplastiques.

11. Structure absorbante (11) selon l'une quelconque des précédentes revendications, caractérisée en ce que la structure contient une couche de renfort en non tissé, en tissu papier, en matière plastique ou en voile par exemple.

12. Article absorbant comme un pansement ou analogue, comprenant une feuille supérieure (12) perméable aux liquides, une feuille inférieure ou de support (13) repoussant les liquides mais perméable à la vapeur, et un corps absorbant (11) enfermé entre lesdites feuilles, caractérisé en ce que le corps absorbant (11) contient une structure absorbante selon l'une quelconque des revendications 4 à 11.

13. Article absorbant selon la revendication 12, caractérisé en ce que la structure absorbante (11) contient essentiellement des fibres de pulpe produite par voie chimio-thermo-mécanique et en ce que la structure contient entre 0 et 10% en poids d'un matériau superabsorbant, valeur calculée par rapport au poids total de la structure à l'état sec.

14. Article absorbante selon l'une quelconque des revendications 12 et 13, caractérisé en ce que la structure absorbante (11) comporte sur une de ses faces une couche de pulpe chimique la recouvrant totalement.
